# EUROPEAN PATENT APPLICATION

(11) **EP 0 704 169 A2**
(43) Date of publication of application: **03.04.1996**
(21) Application number: 95305834.4
(22) Date of filing: 22.08.1995
(51) Int. Cl.: A23L 1/0522, A23L 1/24

(54) **Starch hydrolysis products**

(30) Priority: 27.09.1994 GB 9419392; 16.02.1995 GB 9503017
(71) Applicant: CPC INTERNATIONAL INC., Englewood Cliffs New Jersey 07632-9976 (US)
(72) Inventor: Dea, Iain Cunningham Mutter, Guildford, Surrey, GU1 4DF (GB); Roller, Sibel, c/o School of Applied Science, London SE1 0AA (GB)
(74) Representative: Stevens, Ian Edward

(57) **Abstract**

A starch hydrolysis product suitable for use as a fat and/or oil substitute in food compositions is prepared by heating an aqueous starch suspension containing 10 to 30% by weight starch to 58 to 80°C, treating it with porcine pancreatic α-amylase at from 58 to 63°C and deactivating the enzyme. The starch hydrolysis product is partly soluble in cold water, is not pourable at 90°C, contains partly gelatinised starch granules and exhibits birefringence under crossed-polarised light.

## Description

The present invention relates to starch hydrolysis products which have use as fat and/or oil substitutes in food compositions. It also relates to a method of making the starch hydrolysis products and to the use of such products in food compositions to replace the fats and/or oils conventionally used in such compositions. The invention particularly relates to the use of the starch hydrolysis products in low-fat and zero-fat salad dressings.

Starch hydrolysis products are obtained by the acid or enzymic conversion of starch. The components of a composition, and therefore the properties of a composition, obtained by acid or enzymic conversion of starch depend on the operating conditions employed in the conversion process, particularly the concentration of the reactants, the temperature at which the reaction is conducted and the time for which the reaction is carried out.

Starch hydrolysis products (SHPs), as fat- and oil-substitutes in food compositions, have already been proposed. For instance, GB 1,423,780 discloses a process for the production of starch hydrolysis products having a dextrose equivalent (DE) below 25%, wherein a starch suspension is treated with an α-amylase which is preferably bacterial α-amylase, at a temperature at which the starch granules swell, for from 5 to 30 minutes. The SHPs produced in this way form thermoreversible gels that are reportedly freeze-thaw stable. Recommended food applications using these SHPs include cuttable, low-fat mayonnaise. US 4,510,166 also discloses converted starches for use as fat- or oil-replacement in foodstuffs which are characterised in that the starch has a DE of less than 5 and in that aqueous dispersions thereof at 10 - 50% by weight of starch solids have a hot flow viscosity of at least about 10 seconds at 55°C and are capable of forming gels having a strength of at least 25 g within 24 hours at 4°C. The converted starches may be obtained by heating an aqueous starch slurry and α-amylase until the desired conversion (indicated by DE) is reached.

We have found that salad dressings prepared using the prior art SHPs and no added oil have an unacceptable pasty or gum-like consistency or have other organoleptic properties that are undesirable in such products.

α-Amylases, which catalyse hydrolytic attack of α-D-(1→4) linkages in starch molecules, occur widely in nature; in plants, bacteria and animals. The properties of an SHP produced using α-amylase catalysed hydrolysis of starch have been found to depend on the type of α-amylase used.

We have surprisingly found that SHPs not suffering from the disadvantages of the prior art SHPs may be produced using, as the conversion enzyme, porcine pancreatic α-amylase.

According to the present invention there is provided a process for the production of a starch hydrolysis product which contains partly gelatinized starch granules comprising the steps of
(i) preparing an aqueous starch suspension containing from 10 to 30% by weight (dry substance) of starch;
(ii) heating the aqueous starch suspension to a temperature in the range of from 58°C to 80°C being not significantly higher than the gelatinisation temperature of the starch;
(iii) treating the aqueous starch suspension with a porcine pancreatic α-amylase at a temperature in the range of from 58° to 63°C for a suitable period of time to achieve the required DE and/or molecular weight for the water soluble fraction of the product; and
(iv) deactivating the enzyme.

The SHPs obtainable according to the process of the invention preferably have a DE in the range of from 3 to 12, more preferably 4 to 10, and are partly soluble in cold water, i.e., water of a temperature of 20°C and below. In addition, the SHPs are generally not pourable at 90°C and contain partly gelatinized starch granules observable by microscopic examination and typically exhibit some birefringence under crossed-polarised light. These SHPs can be used to replace, partially or completely, edible fats or oils conventionally employed in food compositions. In particular, they can be used to prepare low- or zero-fat salad dressings, such as mayonnaise, which do not suffer from the textural disadvantages associated with dressings prepared from prior art SHPs.

The starch hydrolysis products of the invention are partly soluble in water at 20°C at a level of 1% by weight and typically comprise from 20% to 60% by weight of a fraction which remains insoluble in water even after boiling. The water soluble fraction of the products preferably shows a peak in its molecular weight profile in the range of from 6 to 30 kDa, preferably in the range of from 8 to 20 kDa. Accordingly, therefore, the present invention also provides a starch hydrolysis product suitable for use as a fat and/or oil substitute in food compositions which is characterised in that it is partly soluble in cold water and its water soluble fraction has a peak in its molecular weight profile in the range of from 6 to 30 kDa.

For the SHPs to be produced according to the present invention, the source of starch subjected to enzymic conversion may be a cereal or root starch, typically selected from barley starch, wheat starch, corn starch, potato starch, tapioca starch, rice starch or sago starch. The starches may be chemically modified (e.g., phosphorus cross-bonded) and may be genetic variations such as waxy corn starch and high amylose corn starch. We have found that SHPs produced from wheat starch according to the present invention are preferred since these have better fat mimetic properties than SHPs produced using the other starch sources.

Starches from different sources may have different gelatinisation temperatures. For instance, not only is it the case that the gelatinisation temperatures of wheat starch and tapioca starch are different but it is also the case that a European wheat starch gelatinizes at a temperature different from the temperature at which an American wheat starch gelatinizes. This difference is, at least partly, due to the difference between the climatic conditions prevailing at the different locations. To take into account these differences, the process of the invention requires the starch, in an aqueous suspension, to be heated to a temperature in the range of from 58° to 80°C but which temperature is not significantly higher than the gelatinisation temperature of the starch being used. This gelatinisation temperature can be determined experimentally. As a result, the starch granules are caused to swell and the starch may become partially gelatinised before or during the onset of enzyme-catalyzed hydrolysis. Preferably, the aqueous starch suspension is heated to a temperature which is slightly below, e.g., 1 - 2°C below, the temperature at which swelling of the starch begins.

The aqueous starch suspension having been heated as described above is then subjected to treatment with a porcine pancreatic α-amylase at a temperature within the range of from 58° to 63°C, preferably 58 - 60°C for a suitable period of time. A typical porcine pancreatic α-amylase that is suitable for use in the present invention is E.C. 3.2.1.1 Type VI-B (Sigma) No. A-3176 which has a reported activity of 25 units/mg solid at pH 6.9 and 13% moisture. One unit of this enzyme is defined as liberating 1 mg of maltose from starch in 3 minutes at 20°C and pH 6.9. Reported β-amylase activity is 7.4 units/mg solid at pH 4.8. The reported pH optimum for this enzyme is 6 - 7 and the enzyme is thermolabile above 50°C. The concentration of the enzyme used in the invention is typically in the range of from 700 to 2100 units/litre.

The period of time suitable for the enzymatic conversion of the starch to give a product having the preferred DE in the range of from 3 - 12 and/or the preferred molecular weight profile having a peak in the range of from 6 to 30 kDa for its water soluble fraction is generally from 5 to 30 minutes and typically from 11 - 15 minutes for a product having a DE in the most preferred range of from 4 to 10.

When the desired DE and/or molecular weight for the water soluble fraction of the product is achieved it is necessary that further hydrolysis of the substrate is prevented by inactivating the enzyme. Inactivation is typically achieved by heating the composition containing the enzyme to a temperature of at least 80°C, preferably 90°C - 95°C.

Following enzyme inactivation, the SHP is cooled prior to use. The SHP may, optionally, be dried by techniques known per se in the art, such as by freeze-drying or spray drying.

The SHPs obtained according to the present invention preferably have a DE in the range of from 3 to 12, more preferably from 4 to 10 and are partly soluble in cold water (i.e., water at a temperature of about 20°C). Typically, the SHPs are not pourable at 90°C. Also the SHPs obtained according to this invention contain partly gelatinized starch granules and generally exhibit birefringence under cross-polarised light indicating the presence of some starch crystals. We have also found that the SHPs of the invention will typically have a break strength in the range of from 5 to 160 g, preferably 5 to 55 g, and a gel strength not greater than 25 g/mm, preferably 2 to 13 g/mm, as measured on a 20% paste of the modified starch (matured for 24 hours), and a peak viscosity of 80 to 1200 BU (Brabender units) measured at a temperature of 51 - 59°C in a Brabender amylograph.

The SHPs may be used as oil- and/or fat-substitutes in food compositions, particularly in 0% oil or 0% fat compositions. They are especially useful as fat mimetics (body-imparting and/or consistency-imparting carriers) in 0% oil salad dressings, such as in low calorie mayonnaises.

### EXPERIMENTAL

SHPs according to the present invention were prepared as described in the following Examples. The enzyme used in the Examples was porcine pancreatic α-amylase (Product A-3176, Type VI-B obtained from Sigma Chemical Company with a reported activity of 25 units/mg solid at pH 6.9). One unit was defined as liberating 1 mg of maltose from starch in 3 min at 20°C and pH 6.9. Reported β-amylase activity was 7.4 units/mg solid at pH 4.8. The reported pH optimum was 6 - 7 and the enzyme was thermolabile above 50°C.

### EXAMPLES 1 TO 9

Wheat starch was dispersed in 0.02 M sodium phosphate buffer (pH 7), with or without 0.02 M sodium chloride added, to give a final starch concentration of 30%. Thus, 9 kg starch was mixed with 21 litres of buffer; 12 kg starch with 28 litres of buffer; and 20 kg starch with 46.7 litres of buffer. The enzyme was dissolved in the same buffer and added to the starch slurry to give the appropriate activity (units/litre of starch slurry) at time zero. The hydrolysis was carried out in a 100-litre surface-scraped, steam-jacketed mixer (Agemore) with a central paddle counter-revolving at 45 rev/min. The temperature of the vessel was controlled by the application of intermittent steam pressure at 5 psig during the reaction and at 15 psig during the enzymic inactivation stage (90 - 95°C) to the steam jacket.

The enzymic hydrolysis of the starch was allowed to take place at 58 - 60°C and the concentration of enzyme added was 700 - 2100 units/litre. The time of hydrolysis (holding time) was varied from 11 - 15 minutes to produce DEs from 3.8 to 9.0. The reaction was terminated by rapid heating of the starch mix to above 90°C to inactivate the enzyme. The hot SHP was poured into metal trays, allowed to cool and blast frozen at -30°C prior to freeze-drying. Once dry, the material was ground in a hammer mill to obtain a powder.

The course of hydrolysis was monitored by periodic removal of samples of the starch mixture which were boiled to inactivate the enzyme, followed by freeze-drying and measurement of reducing sugars by the DNSA method.

Examples of reaction parameters are shown in Table I.

**Table I**

| **Reaction parameters used in the preparation of SHPs** | | | | |
|---|---|---|---|---|
| Example | NaCl | DE | Amount of enzyme (units/1) | Holding time at 59-63°C (min) |
| 1 | Yes | 4.4 | 1400 | 30 |
| 2 | Yes | 4.5 | 1400 | 5 |
| 3 | No | 4.9 | 1400 | 17 |
| 4 | Yes | 6.7 | 1400 | 12 |
| 5 | Yes | 8.4 | 1400 | 19 |
| 6 | Yes | 9.0 | 1400 | 22 |
| 7 | No | 9.2 | 2100 | 22 |
| 8 | No | 9.6 | 2800 | 27 |
| 9 | Yes | 11.0 | 1400 | 30 |

Slurries were prepared by dispersing 80 g of the SHPs in distilled water to make a total volume of 400 ml. The slurries were heated in a Brabender Viscograph (Type 801201) fitted with an eight-prong sample bowl, a seven-prong sensor and a 700-cmg measuring head from 25° to 97°C at 1.5°C per minute. The slurries were held at 97°C for 15 minutes, followed by cooling to 30°C. Viscosities were monitored on a chart recorder throughout the heating and cooling cycle, the recorder being set to zero viscosity between each sample with 400 ml of distilled water in the bowl. Peak viscosity was recorded in Brabender Units.

The viscosifying properties of a range of SHPs prepared are shown in Table II.

**Table II**

| **Viscosities of starch pastes measured in a Brabender Viscograph** (All pastes contained 20% wt/vol sample unless otherwise indicated) | | | | | |
|---|---|---|---|---|---|
| Example No | DE | Starting viscosity (BU) | Pasting temp. (°C) | Peak (BU) | viscosity Temp. (°C) |
| 1 | 4.4 | 100 | 28 | 1163 | 53 |
| 2 | 4.5 | 38 | 27 | 1200 | 57 |
| 3 | 4.9 | 365 | 34 | 615 | 53 |
| 4 | 6.7 | 50 | 25 | 410 | 54 |
| 5 | 8.4 | 60 | 27 | 220 | 55 |
| 6 | 9.0 | 460 | 37 | 460 | 51 |
| 7 | 9.2 | 140 | 35 | 180 | 59 |
| 8 | 9.6 | 65 | 40 | 83 | 58 |
| 9 | 11.0 | 40 | 27 | 350 | 52 |

### The Gelling Properties of SHPs

The SHPs were dispersed in distilled water at a concentration of 20% (wt/vol), heated in a Brabender Viscograph as described above, held at 97°C for 15 minutes and poured, whilst hot, into 120-ml capacity glass jars, the necks of which had been extended with tape. The jars were filled to above the rim, cooled and allowed to mature at 20°C or at 4°C for 24 hours.

The matured starch gels formed a tough skin, which was removed at the rim with a wire cheese-cutter. The gels were analysed with an LFRA/Stevens Texture Analyser fitted with a hemispherically tipped Perspect probe (diameter 13 mm x 3.5 cm). The probe was programmed to penetrate the centre of the gels to a maximum depth of 20 mm at a speed of 0.5 mm/s. The load on the probe was continuously recorded on a chart recorder as a function of penetration distance. The texture analyser was calibrated with a standard 200 g weight such that a response range of 0.200 mV (corresponding to a load of 200 g) was obtained as a full-scale deflection on the chart recorder paper.

The gelling properties of a range of SHPs are shown in Table III.

**Table III**

| **Gel preparations of starch pastes measured in a Stevens/LFRA Texture Analyser** (All gels contained 20% starch unless otherwise indicated) | | | | |
|---|---|---|---|---|
| Example No | DE | Break strength (g) | Elastic limit (mm) | Gel strength (g/mm) |
| 1 | 4.4 | 50.0 | 3.9 | 12.8 |
| 2 | 4.5 | 159 | 6.3 | 25.4 |
| 3 | 4.9 | 42.5 | 3.5 | 12.1 |
| 4 | 6.7 | 38.2 | 3.8 | 10.1 |
| 5 | 8.4 | 24.8 | 4.3 | 5.8 |
| 6 | 9.0 | 54.2 | 4.9 | 11.1 |
| 7 | 9.2 | 8.0 | 3.7 | 2.2 |
| 8 | 9.6 | 5.0 | 2.1 | 2.4 |
| 9 | 11.0 | 46.4 | 5.4 | 8.6 |

### Microscopy

All SHPs prepared contained some partially gelatinised starch grains, as determined by light microscopy. Thus, compact, angular fragments, 30 - 100 µm in size containing tightly packed starch grains were observed. The fragments hydrated slowly in water. Some birefringence was also observed. In contrast, a commercial potato SHP such as Paselli SA2 from Avebe, contained no intact or partially gelatinised granules.

### EXAMPLES 10 - 18

In the following Examples low DE SHPs were prepared from some Latin American starches (Brazilian corn starch, Brazilian wheat starch and Mexican tapioca starch). In these examples the α-amylase was the same porcine pancreatic α-amylase as was used in Examples 1-9.

The wheat starch SHPs were prepared by dispersing the starch (1.2 kg) in 2.8 litres of 10 mM sodium phosphate buffer (pH 7) to give a final starch concentration of 30%. The enzyme was dissolved in 10 ml of the same buffer and then added to the starch slurry at time zero. The starch mixture was stirred using an overhead paddle mixer until the onset of gelatinisation at around 60°C. Following this, the reaction was stirred manually as it was too thick to be stirred by the mixer arrangement. At the end of the reaction period (the 'holding time' ) the temperature of the mixture was raised to 80°C to inactivate the enzyme.

The hydrolysis of the tapioca starch was carried out essentially as above except that the starch slurry was heated to 70°C and allowed to cool (at ambient temperature) to 60°C before addition of the enzyme (the porcine amylase would otherwise be thermally-inactivated). At the end of the reaction period, the temperature of the mixture was raised to 80°C to inactivate the enzyme.

The moisture content of all the SHPs prepared in this study was very similar. Thus, SHPs of Examples 10 and 11 prepared from Brazilian wheat contained 2.4 and 2.6% moisture, respectively; SHPs from Examples 13 and 14 from Mexican tapioca contained 2.8 and 2.1% moisture, respectively; and SHPs from Examples 17 and 18 from Brazilian corn contained 2.9 and 3.1% moisture, respectively.

Table V shows the rheological properties of the Latin American starches and their SHPs as measured in a Brabender Viscograph. The results obtained with Brazilian wheat were similar to those obtained for SHPs prepared from European wheat. For example, SHP from Example 10 with a DE of 4.9 prepared from Brazilian wheat had a peak viscosity of 1300 BU whilst SHP from Example 2 prepared from European wheat with a DE of 4.5 had a peak viscosity of 1200 BU.

Mexican tapioca starch and its products were more viscous than Brazilian wheat starch and its products, as shown in Table V. Although Brazilian corn and its SHPs were not as viscous as the Mexican tapioca and its products, they were more viscous than the Brazilian wheat starch and its products. Therefore, if the Latin American starches were listed in order of decreasing viscosity the order would be: Mexican tapioca Brazilian corn Brazilian wheat. These general trends in viscosity were paralleled in European starches from tapioca, corn and wheat.

The corn starch SHPs were prepared as above except that the starch slurry was heated to 75°C and allowed to cool to 60°C before addition of the enzyme. Problems were experienced during production of the first corn SHP (Example 15) at a concentration of 30%, as the starch thickened to such an extent that stirring became difficult and it was not possible to prepare a homogeneous product. Therefore, subsequent corn SHPs (Examples 16 to 18) were prepared at a concentration of 10%. At the end of the reaction period, the temperature of the mixture was raised to 80°C to inactivate the enzyme.

The course of each hydrolysis reaction was monitored by periodic sampling (5 - 10 ml) of the reaction mixture and boiling for 10 min to inactivate the enzyme. These samples were frozen before freeze-drying in a Lyolab BII laboratory freeze dryer (Life Science Labs. Ltd., Luton, Beds., UK). The bulk of the SHPs at the end of reaction were poured hot into metal trays, cooled at ambient temperature, and frozen before freeze-drying in an Edwards 'Mini-Fast' laboratory/pilot freeze dryer (Model 3400, Edwards High Vacuum, Crawley, West Sussex, UK). Once dry, all the materials were milled to powders, the bulk samples in a hammer mill (Christy and Norris Ltd.), and the time-course samples in a Braun coffee grinder or with a pestle and mortar.

The processing parameters used to prepare the SHPs are summarised in Table IV. The reaction parameters used for the preparation of SHPs from Brazilian wheat were essentially very similar to those used for the manufacture of European wheat SHPs. The reaction parameters used for the preparation of yucca and corn SHPs from Latin America differed from those for wheat SHPs in that each starch substrate was pre-gelatinised prior to the addition of the enzyme. This alteration in methodology was undertaken in light of previous results which showed poor enzyme activity with corn and tapioca substrates without prior pre-gelatinisation.

**Table IV**

| **Reaction parameters used in the preparation of SHPs** | | | | | |
|---|---|---|---|---|---|
| Example | Starch Substrate | Initial Starch concentration (%) | DE | Amount of enzyme (units/1) | Holding Time at 60°C(min) |
| 10 | Brazilian wheat | 30 | 4.9 | 2,100 | 4.5 |
| 11 | Brazilian wheat | 30 | 10.7 | 2,100 | 14.5 |
| 12 | Brazilian wheat | 30 | 11.7 | 2,100 | 20.0 |
| 13 | Mexican tapioca | 30 | 4.6 | 2,100 | 8.0* |
| 14 | Mexican tapioca | 30 | 6.8 | 2,100 | 20.0* |
| 15 | Brazilian corn | 30 | 6.2 | 2,100 | 20.0** |
| 16 | Brazilian corn | 10 | 7.3 | 2,100 | 6.0** |
| 17 | Brazilian corn | 10 | 7.8 | 2,100 | 8.0** |
| 18 | Brazilian corn | 10 | 6.3 | 2,100 | 10.0** |

| | | | | | |
|---|---|---|---|---|---|
| * Starch pregelatinised by heating to 70°C. Enzyme added on cooling to 60°C. | | | | | |
| ** Starch pregelatinised by heating to 75°C. Enzyme added on cooling to 60°C. | | | | | |

**Table V**

| **Viscosities of starch pastes measured in a Brabender Viscograph** | | | | | | | |
|---|---|---|---|---|---|---|---|
| Example | DE | Concentration (% wt/vol) | Starting viscosity (BU) | Pasting temp. (°C) | Peak viscosity Temp | | Viscosity at 30°C (BU) |
| | | | | | (BU) | (°C) | |
| native Brazilian wheat | 0.4 | 10 | 0 | 67 | 385 | 97 | 1200 |
| 10 | 4.9 | 20 | 840* | 35 | 1315 | 55 | 2080 |
| 11 | 10.7 | 20 | 370* | 35 | 755 | 55 | 1130 |
| native Mexican yucca | 0.4 | 10 | 0 | 65 | 2015 | 77 | 1250 |
| 13 | 4.6 | 15 | 600 | 32 | 1910 | 54 | 1705 |
| 14 | 6.8 | 15 | 30 | 32 | 2290 | 54 | 2105 |
| native Brazilian corn | 0.4 | 10 | 0 | 70 | 1075 | 91 | 1950 |
| 18 | 6.3 | 20 | 615* | 38 | 590 | 49 | ND |
| 17 | 7.8 | 15 | 58 | 37 | 63 | 55 | ND |

| | | | | | | | |
|---|---|---|---|---|---|---|---|
| * viscosity decreased with initial stirring | | | | | | | |

Table VI shows the gel properties of the Latin American starches and their SHPs as measured in a Stevens/LFRA Texture Analyser. Again, the SHPs prepared from Brazilian wheat starch had broadly similar gelling properties to those obtained from European wheat starch. For example, SHP from Example 11 with a DE of 10.7 prepared from Brazilian wheat had a gel strength of 6.8 g/mm whilst SHP from Example 9 prepared from European wheat with a DE of 11.0 had a gel strength of 8.6 g/mm. Native Mexican tapioca starch, like its "European" counterpart (tapioca), failed to gel at the concentrations used in this study. However, SHPs from Example 13 (DE = 4.6) and Example 14 (DE = 6.8) prepared from Mexican tapioca produced gels with similar gel strengths as those prepared from either European or Brazilian wheat. The two SHPs prepared from Brazilian corn differed substantially in gel strength: SHP from Example 18 with a DE of 6.3 had a gel strength of 17.5 g/mm whilst SHP from Example 17 with a DE of 7.8 had a gel strength of 8.5 g/mm.

**Table VI**

| **Gel properties of starch pastes measured in a Stevens/LFRA Texture Analyser** | | | | | |
|---|---|---|---|---|---|
| Example | DE | Concentration (% wt/vol) | Break load (g) | Elastic limit (mm) | Gel strength (g/mm) |
| native Brazilian wheat | 0.4 | 10 | 157.6 | 6.13 | 25.7 |
| 10 | 4.9 | 20 | 71.6 | 5.25 | 13.6 |
| 11 | 10.7 | 20 | 30.8 | 4.63 | 6.7 |
| native Mexican yucca | 0.4 | 10 | no gel formed | | |
| 13 | 4.6 | 15 | 37.0 | 6.25 | 5.9 |
| 14 | 6.8 | 15 | 42.8 | 7.13 | 6.0 |
| native Brazilian corn | 0.4 | 10 | 182.4 | 9.25 | 19.7 |
| 18 | 6.3 | 20 | 71.2 | 4.13 | 17.2 |
| 17 | 7.8 | 15 | 20.8 | 4.38 | 4.7 |

### Microscopy

Both differential interference contrast and crossed polarised light showed typical ungelatinised starch grains in all three native starches. In addition, polarised light showed distinct Maltese crosses in the native starches. All the SHPs contained fragments of partially gelatinised starch of different sizes. Some wheat hairs and cell wall material were also observed in the SHPs prepared from Brazilian wheat. Under crossed polarised light, the corn SHPs showed slight birefringency (brightness) but no ungelatinised granules were observed. The SHPs prepared from Mexican tapioca showed slightly more birefringency than corn and so possibly contained some less gelatinised starch. The SHPs from Brazilian wheat showed the most birefringency of all the SHPs prepared in this study and Maltese crosses could be seen in some of the fragments. Overall, all the SHPs prepared in this study contained the sharp fragments of compacted and partially gelatinised starch granules not found in most commercial maltodextrins.

### Product Trials

Zero oil salad dressings were prepared using the SHPs obtained in Examples 10, 11, 13 and 14 as above.

The formulations of the salad dressings (control and 0% oil) were as follows:

| | **Control** 32.5% oil (Actual = 34% fat) due to egg yolks | **Experimental** 0% oil (Actual = 1.5% fat due to egg yolks |
|---|---|---|
| Oil | 32.5 | 0 |
| Water | 49.9 | 76.4 |
| Yolks | 4.9 | 4.9 |
| Starch (National Purity HPC) | 4.6 | 4.6 |
| Sucrose | 3.2 | 3.2 |
| Vinegar (12% acetic acid) | 3.1 | 3.1 |
| Salt | 1.5 | 1.5 |
| Mustard flour | 0.1 | 0.1 |
| Potassium sorbate | 0.1 | 0.1 |
| Xanthan gum | 0.1 | 0.1 |
| Fat mimetic | 0 | 6.0 |

### Method of Manufacture of the Salad Dressings

The following standard method of manufacture was used for all fat mimetics evaluated. Batch sizes of 1 kg were prepared.
i) All the dry ingredients were mixed together in a pan and the water and vinegar were added. The weight of the pan and contents was measured.
ii) The mixture was heated to 90°C and held for 30 seconds at this temperature.
iii) The mixture was then cooled to 20°C and the pan and contents were reweighed. Loss in weight by evaporation was corrected for, by the addition of water.
iv) The egg yolk and oil (if used) was added gradually to the starch paste while the mixture was mixed for a total of 12 minutes using a Silverson mixer.
v) The dressing was distributed into 100-ml sterile jars and stored.

During manufacture of the zero-oil dressings, the SHPs prepared in this study formed pastes that were easily dispersed and smooth and that thickened on heating. The final SHP pastes were generally somewhat thinner than that of the control dressing. The organoleptic assessments of the dressings 24 h after manufacture are shown in Table IV. Both Brazilian wheat and Mexican tapioca SHPs afforded acceptable (in terms of texture) zero-oil dressings when assessed 24 h after manufacture although a very slight starchy off-flavour was occasionally detected.

Following storage for 6 and 14 days, the control dressing containing 32.5% oil had remained unchanged in terms of appearance, texture and flavour. However, the zero-oil dressings prepared with the Brazilian wheat SHPs developed a slightly more gel-like structure with time. Nevertheless, the dressings were considered quite acceptable. Furthermore, the dressing prepared with the more hydrolysed Brazilian wheat SHP (SHP from Example 11, DE = 10.7) appeared to benefit from the thickening effect over the 2-week storage period changing from a pourable dressing with starchy off-flavours at 24 h to a thick dressing similar in texture to the control and improved flavour after 14 days.

Slight improvements with time in texture, mouthfeel and flavour were also observed in the dressings prepared with Mexican tapioca SHPs.

**Table VII**

| **Informal organoleptic assessment of zero-oil dressings 24 h after manufacture** | | | |
|---|---|---|---|
| | Appearance | Texture | Flavour |
| Control (32.5% oil) | White, opaque, thick spoonable, smooth | Smooth, creamy mouth-coating | Creamy, well-rounded |

| Starch Substrate | | | |
|---|---|---|---|
| Brazilian wheat: | | | |
| SHP of Example 10 (DE = 4.9) | Thick, shiny, yellowish, spoonable, slightly gelled | Smooth, thick (but less than control) | Well-balanced, less acidic than control |
| SHP of Example 11 (DE = 10.7) | Smooth, shiny, pourable, not gelled | Bodiless, watery, not thick or creamy | Very slight starchy off-flavour |

| Mexican tapioca: | | | |
|---|---|---|---|
| SHP of Example 13 (DE = 4.6) | Smooth, shiny, spoonable, translucent, thinner than control | Thinner than control, a little watery | Acceptable, less acidic than control |
| SHP of Example 14 (DE = 6.8) | Smooth, shiny, thick, spoonable, slightly gelled | Smooth, thick but thinner than control | Not completely acceptable, starchy off-flavour |

It is of interest to note that the results obtained with the Latin American starches were paralleled by those obtained with European sources of starch. Thus, both European and Latin American SHPs gave satisfactory dressings with a progressive thickening effect over storage time. Slight improvements with time were observed with "European" tapioca SHPs, as with the Mexican tapioca SHPs.

Although certain dressings e.g., that containing SHP from Example 10 (from Brazilian wheat), were considered to have an acceptable texture, all were different from the control in terms of "creamy mouthfeel". Thus, the control dressing was considered "creamy" in a slippery/oily sense whilst the best zero-oil dressings were considered "creamy" on account of their stickiness and slow breakdown in the mouth.

The results of rheological analyses of dressings, summarised in Table VIII confirm the organoleptic observations made in Table VII. Thus, the dressings considered thinnest on sensory assessment e.g., dressings containing Brazilian wheat SHP from Example 11 and Mexican tapioca SHP from Example 13, also had the lowest instrumental viscosities. It would appear that the gel-like structure of the SHP dressings is evaluated as "thickness" by the human assessor whilst the Carri-med instrument gives a measure of viscosity following breakdown of the initial gelled structure. The time dependence of viscosity development in SHP dressings, observed informally during organoleptic assessment, was confirmed instrumentally.

**TABLE VIII**

| **Viscosities (PaS) of dressings containing Latin American SHPs following storage for 24 h, 6 days and 14 days** | | | | | | | | | |
|---|---|---|---|---|---|---|---|---|---|
| | STORAGE TIME | | | | | | | | |
| | 24 h | | | 6 d | | | 14 d | | |
| | Shear rate (s⁻¹) | | | Shear rate (s⁻¹) | | | Shear rate (s⁻¹) | | |
| Starch substrate | 10 | 30 | 100 | 10 | 30 | 100 | 10 | 30 | 100 |
| Control (32.5% oil) | 13.9 | 5.8 | 2.5 | 12.9 | 5.5 | 2.4 | 12.5 | 5.3 | 2.3 |

| Brazilian wheat: | | | | | | | | | |
|---|---|---|---|---|---|---|---|---|---|
| SHP Example 10 (DE = 4.9) | 7.0 | 2.9 | 1.2 | 9.0 | 3.4 | 1.3 | 10.3 | 3.8 | 1.4 |
| SHP Example 11 (DE = 10.7) | 5.2 | 2.5 | 1.2 | 6.2 | 2.7 | 1.2 | 7.3 | 3.1 | 1.4 |

| Mexican tapioca | | | | | | | | | |
|---|---|---|---|---|---|---|---|---|---|
| SHP Example 13 (DE = 4.6) | 5.6 | 2.5 | 1.2 | 6.9 | 2.9 | 1.3 | 7.9 | 3.2 | 1.3 |
| SHP Example 14 DE = 6.8 | 6.6 | 3.1 | 1.4 | 8.1 | 3.5 | 1.5 | 8.7 | 3.7 | 1.6 |

### EXAMPLES 19 - 22

Further samples of SHPs according to the invention were prepared using wheat starch and potato starch. The potato starch was obtained from Roquette Freres (ref: 474095). The wheat starch was also obtained from Roquette Freres (ref: 466539). The enzyme used was porcine pancreatic α-amylase E.C. 3.2.1.1 Type VI-B (Sigma Chemia Co.) No. A-3176 (activity - 25 units/mg solid; 13% moisture).

The reaction was carried out in a Brabender viscograph (801201) fitted with a 700 cm.g sensitivity cartridge. The starch (approx. 30% dry starch content) was suspended in 0.01 M sodium phosphate buffer to a total weight of 498 g and transferred to the Brabender vessel. The enzyme was added to the slurry in the Brabender vessel as a dry powder.

The starch was heated from 25°C to 60°C at a rate of 1.5°C/minute and the temperature was held at 60°C for 20 minutes (15 minutes in one experiment). The starch was heated to 90°C at 1.5°C/minute and then boiled for 15 minutes in a water bath to inactivate the enzyme. The resulting SHPs were allowed to cool and were then frozen and lyophilised in an Edwards MFD 0.5 Pilot scale freeze-drier. The dried SHPs were ground to a fine powder in a coffee grinder.

The reducing sugar content of SHP samples was determined using the dinitrosalicylic acid (DNSA) method (Miller G.L. (1959) "Use of dinitrosalicylic acid reagent for determination of reducing sugar". Analyt. Chem., 31, 426). Results were quoted in dextrose equivalents (DE).

The process conditions are set out in the following Table IX.

**Table IX**

| **Conditions used for the production of SHPs and the resultant DEs** | | | | | |
|---|---|---|---|---|---|
| Ex No. | Starch source | Wet starch content (%) | Enzyme concentration units/g dry starch) | | |
| | | | Dry starch content (%) | Porcine α-amylase | DE |
| 19 | Potato | 30 | 24 | 8 | 8 |
| 20 | Potato | 36 | 28.8 | 7.3 | 7 |
| 21 | Wheat | 30 | 26.1 | 8.1 | 9.7 |
| 22 | Potato | 32.6 | 26.1 | 8 | 7.1 |

It was observed that all samples of SHP prepared as described above were not pourable at 90°C but were semi-solid and had to be transferred to a beaker with a ladle.

### EXAMPLES 23 - 25

Three SHPs were prepared according to the present invention using wheat starch.

Porcine α-amylase was obtained from Sigma Chemical Company Ltd. The enzyme preparation (No. A-6880, Type VI-A) contained sodium chloride and lactose as extenders and had a reported activity of 14 units/mg solid at pH 6.9. One unit was defined as liberating 1 mg of maltose from starch in 3 min at 20°C and pH 6.9. Reported β-amylase activity was 3.3 units/mg solid at pH 4.8. The reported temperature optimum for porcine α-amylase was 37°C.

Wheat starch (1.2 kg) was dispersed in 2.8 litres of 0.06 M sodium phosphate buffer (pH 7) to give a final concentration of 30% starch and a final volume of 4 litres, in a five-litre round-bottom flask on a heating mantle. The enzyme was dissolved in the same buffer and added to the starch slurry to give the appropriate activity units at time zero. The reaction mixture was stirred using a top-loaded paddle mixer until the start of gelatinisation. Following this, the mix was stirred manually and the temperature of the mix was raised.

The enzyme was allowed to hydrolyse the starch at the appropriate temperature for varying lengths of time (holding time) to produce different DEs. The reaction was terminated by rapid heating of the starch mix to 70° - 85°C to inactivate the enzyme. The hot SHP was poured into metal trays, allowed to cool and blast frozen at -30°C prior to freeze-drying (Edwards Mini-Fast Model 3400). Once dry, the material was ground in a hammer mill (Christy and Norris Ltd) to obtain a powder.

The course of selected hydrolysis reactions was monitored by periodic removal of 5 ml of the starch mixture and their addition to 10 ml of methanol to inactivate the enzyme and precipitate the starch. These samples were dried by rotary evaporation.

Details of the processing parameters used to prepare the SHPs are shown below in Table X.

**Table X**

| Example No | DE | Amount of enzyme | | Holding time (min) | Holding temperature (°C) |
|---|---|---|---|---|---|
| | | (g) | units/l | | |
| 23 | 3.8 | 0.20 | 700 | 11 | 58 |
| 24 | 4.5 | 0.20 | 700 | 15 | 59 |
| 25 | 9.0 | 0.60 | 2100 | 11 | 60 |

The SHPs were evaluated as fat mimetics in salad dressings containing 15% oil and 0% oil. The formulations for these dressings and also for a control dressing were as follows:

| Ingredients | Control (32.5% oil) (%) | 15% oil (%) | 0% oil (%) |
|---|---|---|---|
| Hot soluble starch (Purity HPC, National Starch) | 4.6 | 4.6 | 4.6 |
| Egg yolk | 4.9 | 4.9 | 4.9 |
| Vinegar (12% acetic acid) | 3.1 | 3.1 | 3.1 |
| Salt | 1.5 | 1.5 | 1.5 |
| Sucrose | 3.2 | 3.2 | 3.2 |
| Mustard flour | 0.1 | 0.1 | 0.1 |
| Potassium sorbate | 0.1 | 0.1 | 0.1 |
| Xanthan gum | 0.1 | 0.1 | 0.1 |
| Water | 49.9 | 63.6 | 76.4 |
| Oil | 32.5 | 15.0 | 0 |
| SHP | 0 | 3.8 | 6.0 |
| Note: 1) The total fat content of the dressings was 1 - 2% higher than suggested by the level of oil added, owing to the fat content of the egg yolks. | | | |

The dressings prepared were as follows (Table XI)

**Table XI**

| Dressing | Oil Content | SHP Used |
|---|---|---|
| A | 15% | Ex 23 |
| B | 15% | Ex 24 |
| C | 15% | Ex 25 |
| D | 0% | Ex 23 |
| E | 0% | Ex 24 |
| F | 0% | Ex 25 |

The dressings were prepared according to the procedure described above in Examples 10 - 18. Following storage, at 4°C, the dressings were allowed to equilibrate to room temperature for 2 hours and were then subjected to organoleptic assessment. The results are shown in Tables XII - XIV below.

**Table XII**

| **Organoleptic Assessment of Dressings (15% oil) Following Short-Term Storage at 4°C** | | |
|---|---|---|
| Dressing | Appearance | Taste |
| Control (32.5% oil) (Age 6 days) | Smooth, white, shiny, holds shape well. | Very smooth, bland, sticky, pleasant tang, leaves film. |
| A (Age 1 day) | Shiny, smooth, slightly thinner than control. | Very smooth, bland, slightly vinegary, not lumpy. |
| B (Age 1 day) | Shiny,thixotropic, holds shape well. | Very smooth, sticky, bland, slightly starchy flavour but well-liked. |
| C (Age 1 day) | Spoonable, shiny, thinner than B or control. | Very smooth, not grainy, tangy, clear favourite. |

**Table XIII**

| **Organoleptic Assessment of Dressings (15% oil) Following Longer-Term Storage at 4°C** | | |
|---|---|---|
| Dressing | Appearance | Taste |
| Control (32.5% oil) (Age 109 days) | White, opaque, thick, spoonable. | Bland with a vinegary 'kick', filmy, smooth, slightly gummy. |
| A (Age 100 days) | Spoonable, thick, like control, slightly yellow. | Very smooth, slightly vinegary, not grainy, well-liked, second favourite. |
| B (Age 100 days) | Spoonable, very like control | Closest in texture to control, pleasant flavour, first favourite. |
| C (Age 100 days) | Spoonable, a suggestion of 'mousse-like' consistency. | Smooth, slightly thicker than control, vinegar after-kick, third favourite. |

**Table XIV**

| **Organoleptic Assessment of Dressings (0% oil) Following 24 Hour Storage at 4°C** | | | |
|---|---|---|---|
| Dressing | Appearance | Texture | Taste |
| D | Translucent, slightly yellow. | Spoonable, smooth, slightly more gel-like than control. | Sweet, vinegary, pleasant. |
| E | Translucent, almost transparent,slightly yellow. | Thick, smooth, spoonable and slightly gel-like. | Acceptable sweet, tangy, no off flavours. |
| F | Translucent and creamy, slightly yellow. | Spoonable, thick-bodied, creamy, smooth. | Sharp, acceptable no off flavours. |

In addition to the above; 0% oil dressings were prepared according to the above formulation and procedure but using prior art SHPs. These were "Instant N-Oil" obtained from National Starch & Chemical Company (believed to be a product according to US 4,510,166) and potato SHP, produced according to GB 1,423,780 by converting potato starch using bacterial α-amylase and obtained directly from the proprietor of that patent. The results of an organolepic assessment of the dressings prepared using these SHPs are shown below in Table XV.

**Table XV**

| Dressing | Appearance | Texture | Taste |
|---|---|---|---|
| Using "Instant N-Oil" | Orangy-yellow, translucent. | Just pourable, not smooth flowing, not spoonable reasonable body. | Slight off-flavours detected. |
| Potato SHP (Acadamie der Wissenschaften DDR) | Creamy, pale yellow, shiny, translucent. | Thick and spoonable, strongly gelled. | Starchy, floury, very acidic. |

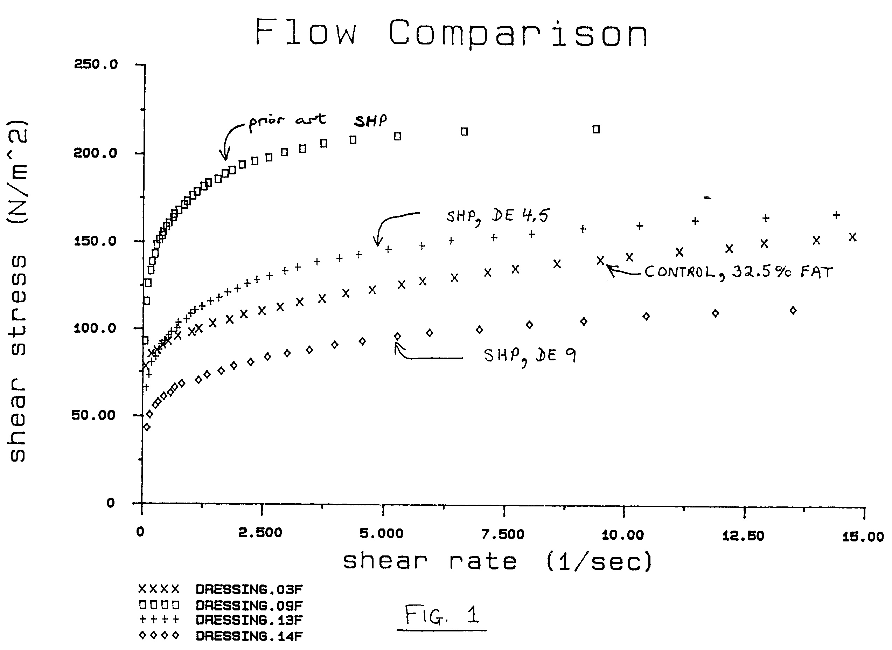

Rheological analyses confirmed the sensory observations. Figure 1 shows the flow curves of shear stress versus shear rate for the control dressing (32.5% oil), and three 0% oil dressings (E and F and the prior art potato SHP) shows that the flow properties of the dressings containing wheat SHPs of the invention were very similar to those of the control dressing; dressing F was a little thinner and dressing E was a little thicker than the control. The shape of the flow curve for the dressing F most closely matched the shape of the control curve. On the other hand, the flow curve for the dressing containing the prior art potato SHP was completely different.

The results in Figure 1 can also be shown in tabular form. Table XVI below shows the dynamic yields and viscosities at three different shear rates. Again, the results show that the rheological properties of the dressing containing the prior art SHP were-very different from those of the control or of the dressings containing SHPs of the invention.

During rheological analysis, it was noted that the dressing containing the prior art potato SHP behaved quite differently in that it tended to break up and fall apart like a gel whilst the other dressings remained smooth and paste-like under shear. Whilst all starch containing dressings tended to thicken with age, the problem was most severe in the dressing containing the prior art potato SHP and least extensive in the dressing F of the invention.

**Table XVI**

| **Rheological characteristics of salad dressings** (All dressings were evaluated after 23 days of storage) | | | | |
|---|---|---|---|---|
| Sample | Dynamic Yield (NM⁻²) | Viscosity (NM^{-2.5}) at shear rates | | |
| | | 0.5s⁻¹ | 2.5s⁻¹ | 10s⁻¹ |
| Control (32.5% oil) | 77.2 | 186 | 44.1 | 14.0 |
| Dressing E | 60.4 | 201.0 | 49.1 | 15.2 |
| Dressing F | 38.9 | 130.0 | 32.4 | 10.2 |
| Prior art potato SHP 0% oil (GB 1,423,780) | 93.4 | 326.7 | 73.2 | 20.4 |

### Molecular Weight Profiling

The molecular weight profile of the water soluble fraction of the products of the invention was investigated for comparison with prior art products.

All samples were made up as 0.1% solutions in 0.1M sodium sulphate by heating to 85°C and filtering through 45 µm membranes. They were eluted from a TSK PW column set (5000/4000/4000) at 45°C at 0.5ml/min. The amount of any insoluble fraction was determined after boiling a 1% mixture of the product in water for 5 minutes by isolating and drying the insoluble material. A range of pullulan standards was made up in cold sodium sulphate solution and eluted under the same conditions.

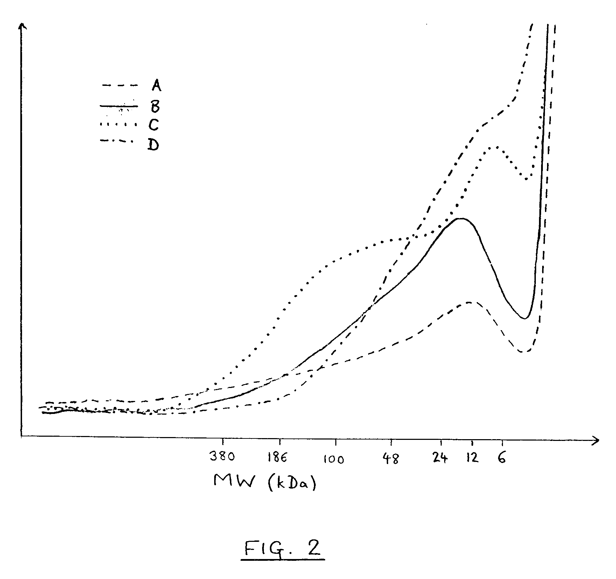

The results of the molecular weight profile investigations are shown in Figure 2 in which the amount of fraction in the eluate is plotted against time, with the time axis converted to a molecular weight scale by reference to the results for the pullulan standards. The plots on the graph are as follows.
- Dashed line A represents the results for a starch hydrolysis product of the present invention made from potato starch in accordance with the method of Examples 1 to 9 but using the porcine α-amylase used in Examples 23 to 25.
- Continuous line B represents the product of Example 7.
- Dotted line C represents the prior art product Paselli SA2.
- Dotted and dashed line D represents a prior art product which is believed to have been produced in accordance with the method disclosed in GB 1,423,780.

All samples contain a salt peak (sodium sulphate) at the end of the run and this coincides in some cases with SHP material of low molecular weight. Since it was not possible to completely resolve the low molecular weight material, namely tri-, di- and monosaccharides from the salt peak, it was not possible to quantify the data to produce molecular weight distributions and averages. Instead, the molecular weight scale, as determined by reference to pullulan standards, is given.

The elution profiles C and D indicate that the prior art materials have quite different molecular weight distributions. Both of these SHPs are completely soluble in water. C has two major components whilst D shows a broad distribution increasing in content as the molecular weight decreases. D also shows a peak at low molecular weight, around 6000 daltons, which coincides with the lower molecular weight component of C. The product illustrated by line D also contains a higher proportion of lower molecular weight material which merges with the salt peak.

The SHPs of the present invention are not completely soluble in water even when heated and the analysis applies to the soluble fraction only. The elution profiles show a broad distribution of molecular weight, increasing in content as the molecular weight decreases and producing a peak at around 12000 daltons in both cases. This peak occurs at a significantly higher molecular weight than the peak in D and there is much less material of lower molecular weight in the products of the invention.

## Claims

1. A process for the production of a starch hydrolysis product which contains partly gelatinized starch granules comprising the steps of
(i) preparing an aqueous starch suspension containing from 10 to 30% by weight (dry substance) of starch;
(ii) heating the aqueous starch suspension to a temperature in the range of from 58°C to 80°C being not significantly higher than the gelatinisation temperature of the starch;
(iii) treating the aqueous starch suspension with a porcine pancreatic α-amylase at a temperature in the range of from 58° to 63°C for a suitable period of time to achieve the required DE and/or molecular weight for the water soluble fraction of the product; and
(iv) deactivating the enzyme.

2. A process according to claim 1, wherein the temperature in step (iii) is about 60°C.

3. A process according to either claim 1 or claim 2, wherein the period of time in step (iii) is from 5 to 30 minutes.

4. A process according to any one of claims 1 to 3, wherein the starch is corn starch, wheat starch, barley starch, tapioca starch, potato starch, rice starch or sago starch.

5. A process according to claim 4, wherein the starch is wheat starch.

6. A process according to claim 5, wherein the temperature to which the aqueous suspension of starch is heated in step (ii) in claim 1 is in the range of from 58° to 63°C.

7. A process according to any one of claims 1 to 6, wherein the enzyme is deactivated by heating the suspension to a temperature of from 90° to 95°C.

8. A process according to any one of claims 1 to 7, wherein the step (iii) of treating the aqueous starch suspension with the α-amylase enzyme is carried out for a period of time to give a product having a water soluble fraction which has a peak in its molecular weight profile in the range of from 6 to 30 kDa.

9. A process according to any one of claims 1 to 8, wherein the step (iii) of treating the aqueous starch suspension with the α-amylase enzyme is carried out for a period of time to achieve a DE in the range of from 3 to 12.

10. A process according to any one of claims 1 to 9 which comprises the further step of subjecting the suspension, after the enzyme has been deactivated, to drying.

11. A starch hydrolysis product suitable for use as a fat and/or oil substitute in food compositions characterised in that it is obtainable by a process according to any one of claims 1 to 10, said product being partly soluble in cold water, being not pourable at 90°C, containing partly gelatinized starch granules and exhibiting birefringence under crossed-polarised light.

12. A starch hydrolysis product according to claim 11 capable of forming aqueous gels at a concentration of 20% (wt/v) having a gel strength of not more than 25 g/mm in 24 hours at 4°C.

13. A starch hydrolysis product according to claim 11 or claim 12 having a DE in the range of from 3 to 12.

14. A starch hydrolysis product suitable for use as a fat and/or oil substitute in food compositions characterised in that it is partly soluble in cold water and its water soluble fraction has a peak in its molecular weight profile in the range of from 6 to 30 kDa.

15. Product as claimed in claim 14, wherein the peak in the molecular weight profile is in the range of from 8 to 20 kDa.

16. A food composition containing one or more food ingredients and a body-imparting and/or consistency-imparting carrier characterised in that the body-imparting and/or consistency-imparting carrier comprises a starch hydrolysis product according to any one of claims 11 to 15.

17. A food composition according to claim 16 which contains no oil.

18. A food composition according to claim 16 or claim 17 which is a salad dressing.
